(19) Europäisches Patentamt
European Patent Office
Office européen des brevets

(11) **EP 4 082 496 B1**

(12) **EUROPEAN PATENT SPECIFICATION**

(45) Date of publication and mention
of the grant of the patent:
**25.03.2026 Bulletin 2026/13**

(21) Application number: **22169157.9**

(22) Date of filing: **06.07.2020**

(51) International Patent Classification (IPC):
*A61F 13/15* (2006.01)  *A61F 13/532* (2006.01)
*A61F 13/534* (2006.01)  *A61F 13/539* (2006.01)
*B05C 19/04* (2006.01)

(52) Cooperative Patent Classification (CPC):
**A61F 13/5323; A61F 13/15634; A61F 13/15658;
A61F 13/534; A61F 13/539;** A61F 2013/53463;
A61F 2013/53472; A61F 2013/53908

(54) **ABSORBENT ARTICLE WITH IMPROVED CORE**

SAUGFÄHIGER ARTIKEL MIT VERBESSERTEM KERN

ARTICLE ABSORBANT À ÂME AMÉLIORÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**02.11.2022 Bulletin 2022/44**

(62) Document number(s) of the earlier application(s) in
accordance with Art. 76 EPC:
**20184283.8 / 3 936 098**

(73) Proprietors:
• **Ontex BV**
**9255 Buggenhout (BE)**
• **Ontex Group NV**
**9320 Erembodegem (BE)**

(72) Inventors:
• **PANNWITT, Stefanie**
**56727 Mayen (DE)**
• **GRANADO, Martín**
**56727 Mayen (DE)**

• **GARAJ, Norbert**
**56727 Sankt Johann (DE)**
• **LAMBERTZ, Christina**
**56566 Neuwied (DE)**
• **MAILINGER, Christel**
**65604 Elz (DE)**
• **WEBER, Ainas**
**53474 Bad Neuenahr-Ahrweiler (DE)**
• **HEEGE, Thomas**
**56761 Düngenheim (DE)**
• **INGENFELD, Björn**
**53115 Bonn (DE)**

(74) Representative: **Macchetta, Andrea**
**Ontex BV**
**Korte Keppestraat 21**
**9320 Erembodegem-Aalst (BE)**

(56) References cited:
**EP-A1- 2 679 210**    **WO-A1-2016/178144**
**WO-A1-2019/182526**    **US-A1- 2006 184 146**
**US-A1- 2016 045 379**    **US-A1- 2016 296 386**
**US-A1- 2019 159 946**

EP 4 082 496 B1

## Description

## TECHNICAL FIELD

The disclosure relates to absorbent articles such as disposable absorbent articles, preferably selected from the group consisting of diapers (whether for baby or adults), pants (whether for baby or adults), pantiliners, briefs, sanitary napkins, and combinations thereof.

## BACKGROUND

Absorbent articles comprising different channel structure designs for enhancing liquid distribution and maximising the use of the core have been developed.

WO2012/170778 (Rosati et al., see also WO2012/170779, WO2012/170781 and WO2012/1708008) discloses absorbent structures that comprise superabsorbent polymer, optionally a cellulosic material, and at least a pair of substantially longitudinally extending channels. The core wrap can be adhesively bonded through the channels to form a channel bond. The channel bonds may be permanent, so that their integrity is at least partially maintained both in dry and wet state. As the absorbent structure absorbs liquid and swells, the absorbent structure takes a three-dimensional shape with the channels becoming visible. The channels provide improved fit and/or liquid acquisition/-transportation, and/or improved performance throughout the use of the absorbent structure.

Further improvements in channel geometries for better core utilisation and liquid distribution are described in EP3342386 describing an absorbent core comprising substantially continuous zones of one or more high fluid distribution structures and discontinuous zones of fluid absorption structures surrounding the one or more high fluid distribution structures, wherein the one or more high fluid distribution structures are arranged to distribute fluid across the absorbent core at a speed that is faster than the speed of fluid distribution across the absorbent core by said discontinuous fluid absorption structures, and wherein said continuous zones extend along a path that is substantially parallel to at least a portion of the perimeter of the core, said portion of the perimeter of the core comprising at least a portion of the sides of the core and one of the ends of the core.

Investment in improved processes for providing channelled absorbent articles has been made. For example WO2018/172860 describes a method for forming an absorbent pad comprising a first layer, a second layer and an absorbent material interposed between the first and the second layer and arranged according to a spreading pattern M1 having at least one channel which is free of absorbent material, comprises a step of feeding a first web (NW1), intended to form the first layer of the pad; a step of feeding a second web (NW2), intended to form the second layer of the pad; a step of spreading the absorbent material on the first web (NW1) according to the spreading pattern M1; a step of joining the first and second webs (NW1, NW2), a step of removing any absorbent material that may be present in the channel.

Another example is EP3453368 that describes a method for manufacturing an absorbent article, said method comprising: a. applying a first binder in a first area on a first side of first sheet material; b. applying a second binder in a second area on a first side of second sheet material; c. applying an absorbent material on the first side of the first sheet material; d. attaching the first sheet material to the second sheet material with the first sides facing each other, such that at least one attachment zone is formed; wherein one of the first sheet material and the second sheet material is a top core wrap sheet material and the other is a back core wrap sheet material; and the first area is arranged at a distance from the intended position of the at least one attachment zone, wherein the first area and the second area are substantially complementary after the step of attaching the wrap sheets to each other.

Typically absorbent cores comprise absorbent material that is freely distributed within the core wrap that encloses such absorbent material therein. It has been observed that, due to absence of immobilisation means, the material is prone to collapse during the stress induced by the movement of the wearer. Although core integrity is generally improved for channelled products as some barrier for movement of the absorbent material is created, there is still a need for significant use of adhesive and/or mechanical bonding to achieve reasonable core integrity. It has been further found that degree of immobilization increases the more that absorbent material is maintained (or spatially constricted/restricted) into compartments. In particular, the inventors have found that spatial constriction in the thickness direction is particularly beneficial to restricting movement of the absorbent material, especially when the absorbent material comprises a mixture of cellulose fibers (i.e. fluff pulp) and superabsorbent polymer particles (i.e. SAP).

EP2679210 A1 describes an absorbent article comprising a wearer-facing topsheet, a backsheet and an absorbent core disposed between the topsheet and the backsheet, characterized in that the absorbent core comprises: a first absorbent layer comprising a first substrate, a layer of first superabsorbent polymer particles deposited on the first substrate, and a fibrous layer of thermoplastic adhesive material covering the layer of first superabsorbent polymer particles; a second absorbent layer, the second absorbent layer comprising a second substrate and a mixed layer deposited on the second substrate, the mixed layer comprising a mixture of second superabsorbent particles and cellulosic fibers, the first absorbent layer and the second absorbent layer being combined together such that at least a portion of the fibrous layer of thermoplastic adhesive material of the first absorbent layer contacts at least a portion of the mixed layer of the second absorbent layer, and wherein the first absorbent layer is placed closer to the topsheet than the second layer.

There is therefore a need for absorbent articles and methods of making that can improve core stability whilst yet being cost effective and provide enhanced liquid handling properties.

There is also, or alternatively, still a need to provide improved absorbent articles comprising channel forming areas and methods of making. In particular there is a need for absorbent articles comprising channel forming areas and methods of making that allow for strong bonding strength and resilience in the channel forming areas whilst retaining optimal core stability and/or improving core flexibility and/or liquid intake permeability therethrough.

## SUMMARY

In a first aspect, the disclosure relates to an absorbent article as described in Claim 1 and Claims dependent therefrom.

## BRIEF DESCRIPTION OF THE FIGURES

Fig. 1A-D Illustrates absorbent articles according to embodiments according to the present disclosure.

Fig. 2A-C Illustrates absorbent articles according to embodiments according to the present disclosure.

Fig. 3A-C Illustrates absorbent articles according to embodiments according to the present disclosure.

Fig. 4A-C Illustrates absorbent articles according to embodiments according to the present disclosure.

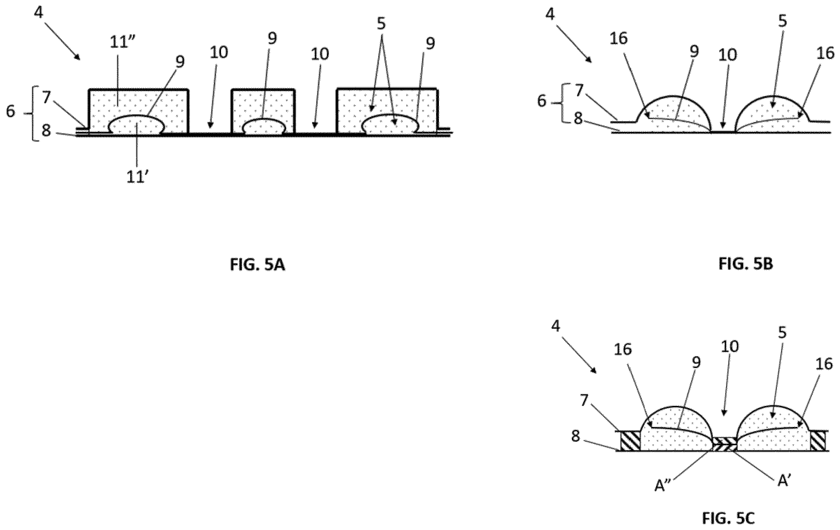

Fig. 5A-C schematically illustrate a cross-section of absorbent cores used in absorbent articles according to embodiments of the present disclosure.

Fig. 6 schematically illustrates a cross-section of absorbent cores used in absorbent articles according to embodiments of the present disclosure and showing different bonding positions b.

Fig. 7A-D schematically illustrate adhesive patterns according to embodiments of the present disclosure.

Fig. 8 schematically illustrates an apparatus for use in methods of making articles, according to an embodiment of the present disclosure.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements, such as a laminate.

"Laminate" refers to elements being attached together in a layered arrangement.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 $\mu$m or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be preformed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc. The recitation of numerical ranges by endpoints includes all numbers and fractions subsumed within that range, as well as the recited endpoints unless otherwise stated.

"Thickness or caliper" herein are used interchangeably, and refer to the caliper typically measured as follows: at 0.5 kPa and at least five measurements are averaged. A typical testing device is a Thwing Albert ProGage system. The diameter of the foot is between 50 mm to 60 mm. The dwell time is 2 seconds for each measurement. The sample is to be stored at 23 + 2°C and at 50 + 2% relative humidity for 24 hours with no compression, then subjected to the fabric thickness measurement. The preference is to make measurements on the base substrate before modification, however, if this material is not available an alternative method can be used. For a structured substrate, the thickness of the first regions in between the second regions (displaced fiber regions) can be determined by using a electronic thickness gauge (for instance available from McMaster-Carr catalog as Mitutoyo No 547- 500). These electronic thickness gauges can have the tips changed to measure very small areas. For example, a blade shaped tip can be used that is 6.6mm long and 1mm wide. Flat round tips can also be inserted that measure area down below 1.5mm in diameter. For measuring on the structured substrate, these tips are to be inserted between the structured regions to measure the as-produced fabric thickness. The pressure used in the measurement technique cannot be carefully controlled using this technique, with the applied pressure being generally higher than 0.5kPa.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

"Wetlaid" as used herein means nonwovens obtained by a process similar to paper manufacturing. The difference lies in the amount of synthetic fibres present in a wetlaid nonwoven. A dilute slurry of water and fibres is deposited on a moving wire screen, where the water is drained and the fibres form a web. The web is further dewatered by pressing between rollers and dried. Impregnation with binders is often included in a later stage of the process.

"Airlaid" as used herein means a process wherein fibres, which are typcially relatively short, are fed into a forming head by an airstream. The forming head assures a homogeneous mix of all fibres. By air again, a controlled part of the fibre mix leaves the forming head and is deposited on a moving belt, where a randomly oriented web is formed. Compared with carded webs, airlaid webs have a lower density, a greater softness and an absence of laminar structure.

"Adhesive" typically means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

As used herein, the term "transverse" or "lateral" refers to a line, axis, or direction which lies within the plane of the absorbent article and is generally perpendicular to the longitudinal direction.

"Dry-state" refers to the condition in which an absorbent article has not yet been saturated with exudates and/or liquid.

"Wet-state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

"Spunlaced" as used herein refers to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 $\mu$m, preferably between 300 to 600 $\mu$m, more preferably between 400 to 500 $\mu$m. Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present disclosure in an amount up to about 90% by weight.

By "substantially", it is meant at least the majority of the structure referred to.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body (e.g. the face) of the wearer during ordinary use, while the "outward", "outward-facing" surface is on the opposite side, and is intended to be disposed to face away from the wearer's body during ordinary use.

Embodiments of the articles and processes according to the disclosure will now be described. It is understood that technical features described in one or more embodiments maybe combined with one or more other embodiments without departing from the intention of the disclosure and without generalization therefrom.

THE ABSORBENT ARTICLE

As illustrated in the figures, in one aspect the disclosure relates to an absorbent article (1) comprising: a liquid permeable topsheet (2), a liquid impermeable backsheet (3), and an absorbent core (4) positioned between said topsheet (2) and backsheet (3), wherein the absorbent core (4) comprises an absorbent material (5), said absorbent material comprising cellulose fibers and/or superabsorbent polymers, and wherein said absorbent material is contained within at least one core wrap substrate (6) enclosing said absorbent material therein, wherein the absorbent core further comprises an intermediate layer (9) positioned between a top layer (7) of said core wrap and a bottom layer (8) of said core wrap such that said absorbent material (5) is disposed between said top layer (7) and said intermediate layer (9) and/or between said bottom layer (8) and said intermediate layer (9) preferably wherein at least the absorbent material comprised between said intermediate layer (9) and said bottom layer (8) comprises a mixture of cellulose fibers and superabsorbent polymer particles (typically comprising more than 10%wt, preferably from 15% to 30%, of cellulose fibers by weight of the absorbent material), and wherein said top layer (7), said bottom layer (8), and said intermediate layer (9) are joined together to form one or more channel forming areas (10) substantially free of said absorbent material. Advantageously, this arrangement allows to improve core integrity in an effective way and at low cost.

Preferably, the absorbent material (5) comprises a mixture of superabsorbent polymer particles and cellulose fibers (preferably at the amounts described hereinbelow) at least in one, preferably both, of: (i) between said top layer (7) and intermediate layer (9); and (ii) between said intermediate layer (9) and said bottom layer (8). Most preferably wherein said mixture comprises more cellulose fibers between said intermediate layer (9) and said bottom layer (8) than the amount of cellulose fibers comprised between said top layer (7) and intermediate layer (9). Advantageously this allows to provide better rewet performance and provide an improved dryness perception upon wetting.

In an embodiment, the absorbent material comprises first superabsorbent polymer particles between said top layer (7) and intermediate layer (9); and second superabsorbent polymer particles between said intermediate layer (9) and said bottom layer (8); wherein said first and second superabsorbent polymer particles have different properties, said properties selected from the group consisting of absorption speed (seconds); absorbency under load (AUL) at 0.7 psi (g/g); and combinations thereof. Preferably wherein the second superabsorbent polymers have a lower vortex and/or absorbency under load than said first superabsorbent polymers. More preferably wherein: (a) the first absorbent polymer particles have an AUL of more than 18 g/g, preferably from 20 g/g to 50 g/g; and/or a vortex of more than 50s, preferably from 60s to 90s; and/or (b) the second absorbent polymer particles have an AUL of less than 18 g/g, preferably from 5 g/g to 15 g/g; and/or a vortex of less than 50s, preferably from 15s to 45s. Advantageously this allows for improved liquid absorbtion performance.

In a preferred embodiment, at least the second superabsorbent polymer particles comprise biocompatible and/or biodegradable superabsorbent polymer particles such as clay, gelatin, and/or sugar comprising particles. In an embodiment, the biocompatible, biodegradable, macromolecular water-absorbent hybrid material (WAHM), has a three-dimensional configuration with intermolecular covalent bonds and containing free functional groups selected from OH, SH, NH2 and COOH, said polymer being formed by polymer-polymer intercoupling reaction between a natural water-soluble polymer A or its derivatives having a molecular weight between 20,000 and 300,000 Da, and a synthetic polymer B in an adequate ratio between 1 and 50% from dried mixture (A+B), wherein the natural polymer A is selected from: amphoteric reactants, partially denatured or chemically modified natural polymer, that dissociates in water to form both anions and cations, and which can undergo polymer-polymer intercoupling reactions, and wherein: synthetic polymer B is a linear or branched reactive synthetic copolymer having a molecular weight of 50,000-500,000 Da derived from a vinyl monomer and an ethylenically unsaturated monomer, said copolymer having a backbone with polymeric subunits Rn and Rf, wherein R represents a subunit covalently bonded to the polymer backbone, n represents non-reactive chemical functional groups and r represents reactive chemical functional groups, as generally exemplified in US20090306290, in particular examples 1 to 8. Indeed without wishing to be bound by theory, such SAPs although beneficial for the environment show higher rewet behaviour, it is thus desirable to include such materials below the intermediate layer (and above the bottom layer (8)) in order to ensure improved dryness on the skin surface.

The cores described herein may comprise more than one intermediate layers (9). Preferably wherein each intermediate layer (9) comprises absorbent material on both a garment-facing surface and body-facing surfaces thereof. Tyically wherein all intermediate layers (9) are contained and/or sandwiched between the top layer (7) and bottom layer (8). Advantageously, a multi-layer core can be made which can further compartmentalise the absorbent material along the thickness direction and hence providing core stability as well as modulating liquid flow through the core with different SAP/fluff concentrations and SAP grades.

When a plurality of intermediate layers (9) are comprised, it is preferable that at least two of said layers (9) have a different width (generally taken along the transversal axis that runs perpendicular to the longitudinal axis y), preferably wherein said width is less than the width of the top and/or bottom layers (7, 8). Preferably, the intermediate layer being closest to the top layer (7) having the greatest or smallest, preferably the smallest, width compared to other intermediate layers. This arrangement allows for a multi-layer stacked core that however limits stiffening the core structure especially upon wetting thus improving the overall comfort as well as providing absorbancy benefits. Moreover, having an intermediate layer closest to the top layer being smallest in width ensures reduced risk of rewet on the topsheet side of the article.

When the core comprises a plurality of intermediate layers (9), the width thereof of each said intermediate layers is typically less than that of the top and bottom layers (7, 8) such that the top and bottom layers may be joined to each other at least along a portion of the perimeter thereof to sandwich said intermediate layers (and absorbent material) therebetween. The plurality of intermediate layers (9) may consist of the same nonwoven having different basis weight in $g/m^2$, or may consist of different materials such as different nonwovens or films as described herein below.

The basis weight of the intermediate layer (9) is greater or equal to the basis weight of the top and/or bottom layers (7, 8). Typically the top and/or bottom layers (7, 8) have a basis weight of from 5 gsm to 45 gsm, preferably 7 gsm to 40 gsm, more preferably 8 gsm to 35 gsm, even more preferably from 9 gsm to 30 gsm, even more preferably from 10 gsm to 28 gsm.

The intermediate layer (9) has a basis weight of from 15 gsm to less than 80 gsm ($g/m^2$), preferably from 15 gsm to 75 gsm, more preferably from 20 gsm to 70 gsm, more preferably from 25 gsm to 65 gsm, more preferably from 30 gsm to 60 gsm, more preferably from 35 gsm to 55 gsm, even more preferably from 40 gsm to 50 gsm. Advantageously this arrangement allows for good mechanical integrity that supports overall core integrity whilst enhancing liquid distribution whilst eliminating unnecessary cost of higher basis weights. Moreover, increasing the basis weight more that the described upper limits leads to, during use, an increase risk of inadvertent release of the bonds joining the top/bottom core wrap layers to the intermediate layer due to an increase risk of absorbent material (especially fluff) contamination that weakens the bonding strength.

The intermediate layer has a thickness that is greater or equal, preferably greater, than the thickness of the top and/or bottom layers (7, 8). Preferably the intermediate layer (9) has a thickness of from 0.5mm to 8mm, preferably from 1mm to 7mm, more preferably from 2mm to 6mm, even more preferably from 3mm to 5mm, even more preferably between and not including 3mm and 5mm. Advantageously this allows to have sufficient bulk for improved liquid transport within the core whilst ensuring adhesion of the layers is not negatively impacted.

In an embodiment, the intermediate layer (9) comprises a structured fibrous web that is thermally stable; the structured fibrous web comprising a first surface and a second surface, a first region and a plurality of discrete second regions disposed throughout the first region, the second regions form discontinuities on the second surface and displaced fibers on the first surface wherein at least 50% and less than 100% of the displaced fibers in each second region are fixed along a first side of the second region and separated proximate to the first surface along a second side of the second region opposite the first side forming loose ends extending away from the first surface, wherein the displaced fibers forming loose ends create void volume for collecting fluid, and wherein the fibers of the structured fibrous web are formed from a thermoplastic polymer comprising a polyester, wherein the structured fibrous web comprises a bio-based content of 10% to 100% using ASTM D6866-10, method B, in another embodiment a bio-based content of 25% to 75% using ASTM D6866-10, method B. In such embodiments, it is preferred that the thickness of at least said first regions in between the second regions (displaced fiber regions) is of from 2mm to 10mm, preferably from 3mm to 9mm, more preferably from 4mm to 8mm, even more preferably from 5mm to 7mm, even more preferably between and not including 5mm and 7mm.

In an embodiment, the intermediate layer (9) is joined, preferably directly, to the top layer (7) and the bottom layer (8) of the core wrap substrate (6) such that said top layer (7) is joined to a body-facing surface of the intermediate layer (9) and the bottom layer (8) is joined to a garment-facing surface of the intermediate layer (9), typically at least within the channel forming areas (10).

Preferably, the absorbent core (4) comprises a plurality of clusters (11) of absorbent material (5) wherein more than one, preferably the majority, even more preferably each, of said clusters is circumscribed by said channel forming areas (10), and wherein each said cluster is separated from the neighboring cluster by said channel forming areas (10). Advantageously such clusters form defined compartments for absorbent material that is retained and prevented from dislodging during use especially when wetted.

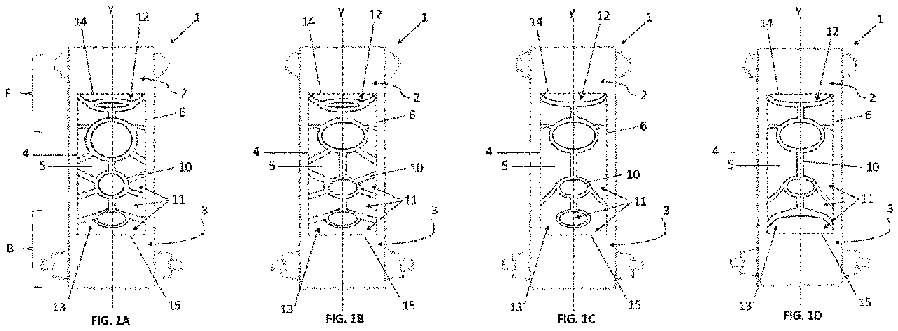

In a highly preferred embodiment, and as exemplified for example in Figs. 1A-D, the absorbent core (4) comprises a plurality of clusters (11) disposed on said core such to form a substantially irregular pattern (generally meaning that the pattern has less than two axis of symmetry and typically is not symmetrical on both the longitudinal axis y and the transverse axis perpendicular thereto generally in a planar view such as as shown in Figs.1A-D). Preferably wherein said pattern has only one axis of symmetry, typcially being the longitudinal axis (y). Advantageously this allows for better usage of the core in terms of absorbent capacity upon wetting.

In a preferred embodiment, more than 15%, preferably more than 20%, even more preferably from 25% to 90%, even more preferably from 30% to 80%, of said clusters have at least one of a different shape; a different size (such as total perimeter or total surface area); and combinations thereof, typically when looked at in a planar view (as exemplified in Figs.1A-D). The percentages typically counted as the total number of clusters having such different shape and/or size divided by the total number of clusters in the absorbent core. This arrangement has the advantage of not only providing better usage of the core surface in terms of absorbancy but further upon wetting and swelling of the absorbent material it allows for graded and/or differentiated/non-uniform swelling that reduces stiffening effects compared to regular/even swelling observed when clusters are substantially identical in shape and/or size, thus providing an overal benefit in terms of fit and comfort even when the article is soiled.

In an embodiment, the top layer (7), the bottom layer (8), and the intermediate layer (9) are joined together by one or more adhesives. Alternatively or in addition, the top layer (7), the bottom layer (8), and the intermediate layer (9) are joined together by one or more mechanical bonds selected from the group consisting of ultrasonic bonds, thermal bonds, pressure bonds, and combinations thereof. When adhesive and mechanical bonds are combined, they are preferably applied in different portions of the channel forming areas (10) so that no same portion comprises both adhesive and mechanical bond. Advantageously this allows to modulate the stiffness and degree of wet integrity of the clusters that may be thus arranged to release upon swelling when wetted in some regions of the absorbent core.

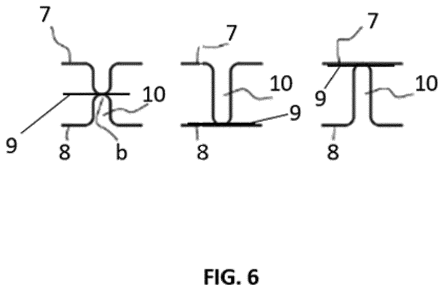

In an embodiment, as illustrated in Fig. 6, the top, intermediate, and bottom layers (7, 9, 8) are joined together in the channel forming areas (10) at a bonding position (b) that may be substantially in the middle, bottom or top of a thickness of the absorbent core.

In an embodiment, the channel forming areas (10) are interconnected such that they extend both along a longitudinal axis (y) and a transversal axis, running substantially perpendicular to the longitudinal axis (y). This allows liquid to flow through the channels long both the longitudinal direction and transverse direction of the absorbent core and be absorbent by neighboring clusters as it makes its way along the channel, hence making more effective use of the core via proper liquid distribution across its entire surface.

In an embodiment, the absorbent core (4) comprises a first terminal edge cluster (12) and/or a second terminal edge cluster (13) positioned on opposite front and/or back transverse edges (14, 15) of the core respectively (generally respectively positioned on the front (F) and/or back (B) of the absorbent article), and arranged such that channel(s) formed by the channel forming areas (10) are separated from said front and/or back transverse edges (14, 15) by said first terminal edge cluster (12) and/or said second terminal edge cluster (13) respectively. Advantageously, these behave like barriers to leakage by ensuring a large space of absorbent material to be present along such terminal edges so that liquid is quickly absorbed before reaching the edge and hence reducing the likelihood of leakage.

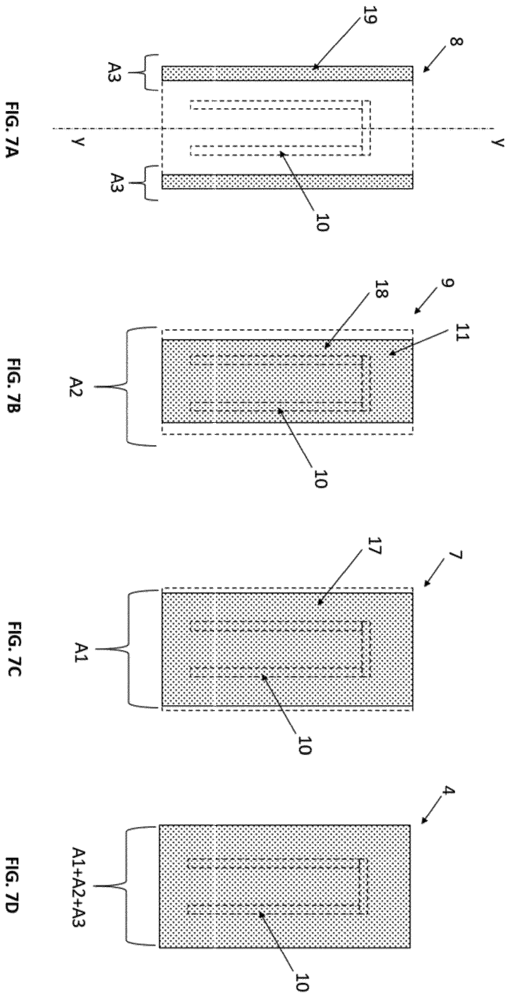

In an embodiment, as illustrated in Fig. 7A-D, the top layer (7) comprises a first adhesive (17) arranged to define a first adhesive area (A1), the intermediate layer (9) comprises a second adhesive (18) arranged to define a second adhesive area (A2), and the bottom layer (8) comprises a third adhesive (19) arranged to define a third adhesive area (A3), wherein said first adhesive area (A1) and the second adhesive area (A2) are greater than the third adhesive area (A3), and preferably wherein said first, second, and third adhesive areas (A1, A2, A3) comprise one or more adhesive stripes. Preferably, wherein the first adhesive layer (A1) is greater or equal to the second adhesive area (A2), and preferably at least one of said adhesive stripes of the first adhesive area (A1) and the second adhesive area (A2) overlaps said channel forming areas (10), and wherein at least one, preferably at least two, of said adhesive stripes of the third adhesive area (A3) is positioned outboard of said channel forming areas (10). Preferably, wherein the third adhesive area (A3) consists of a plurality of spaced apart adhesive stripes wherein at least one, preferably at least two, more preferably at least three, even more preferably from 4 to 7, of said adhesive stripes is positioned outboard and/or inboard of the channel forming areas (10). Advantageously this allows for optimal attachment of the layers in the channel forming areas whilst limiting contamination (e.g. absorbent material sticking into the channel regions prior to joining the other layers in said channel forming areas).

In an embodiment, the first and/or second adhesive areas (A1,A2) comprise a substantially uniform single stripe of adhesive arranged to overlap at least a substantial portion of the channel forming areas (10).

The intermediate layer (9) is selected from the group consisting of a nonwoven, film, and combinations thereof, preferably a nonwoven selected from the group consisting of wetlaid, airlaid, carded, spunbond, meltblown, carded thermobonded, air-through-bonded, spunlaced, tissue and combinations thereof, more preferably a nonwoven selected from the group consisting of carded thermobonded, air-through-bonded, spunlaced, and combinations thereof, most preferably a nonwoven selected from the group consisting of air-through-bonded, spunlaced, and combinations thereof, most preferably a spunlaced nonwoven. Such nonwovens can comprise synthetic or natural fibers. Synthetic fibers are typically selected from the group consisting of polyethylene (PE), polypropylene (PP), polylactic acid (PLA), and mixtures thereof. Natural fibers are typically cellulosic and may be selected from the group consisting of cotton, rayon, micro-fibril cellulose (MFC), derivatives thereof, and mixtures thereof. Most preferred are spunlaced nonwovens generally comprising natural fibers. A particular advantage of spunlaced nonwovens is not only its environmentally friendly impact but further this material normally soaks up liquid and thus is generally seen in the industry as undesirable in view of the rewet disadvantages in absence of other technologies to limit this drawback, in this case however this is turned into an advantage as the layer behaves as a liquid distribution layer within the core itself and thus promoting further distributed absorption by the absorbent material being present both above and below such layer.

In an embodiment, the top layer (7) and bottom layer (8) are the same or different, preferably different, from the intermediate layer (9), and are preferably a nonwoven selected from the group consisting of spunbond, meltblown , carded thermobonded, and combinations thereof.

In an embodiment, the intermediate layer (9) has a width, extending along an axis substantially perpendicular to a longitudinal axis (y), that is less than or equal to a width, extending along an axis substantially perpendicular to a longitudinal axis (y), of the top and/or bottom layers (7,8) of the core wrap. Especially when the width is less than that of the core wrap core stability is retained whilst limiting cost and use of raw material.

Preferably, the intermediate layer comprises at least one, preferably two, free end (16) that is not joined to the top layer (7) and/or bottom layer (8), preferably two oppositely disposed free ends such that each free end is substantially surrounded by absorbent material, preferably said absorbent material being disposed on a garment facing surface, skin facing surface, and lateral edge, of each free end. Advantageously, this allows the intermediate layer to act not only as core integrity system but also further as a liquid distribution layer with the free ends being free to transport liquid directly in middle of the absorbent material and increasing the surface area in contact thereto for optimal liquid transport.

In an embodiment, the top layer (7) is, preferably directly, joined to the bottom layer (8) at one or more positions being outboard of the intermediate layer (9) and typically at the lateral or longitudinal edges forming the perimeter of the absorbent core and extending substantially parallel to a longitudinal axis (y). Said edges may also be folded over themselves to form a C-fold (typically such that folded flaps are in contact twith the body-facing surface of the top layer (7) or the garment-facing surface of the bottom layer (8) and can be held in position via adhesive and/or mechanical bonding). Advantageously, this allows to reduce the risk of absorbent material leaking out of the core during use.

In an embodiment, the intermediate layer is bonded substantially only in the channel forming areas (10), and typically not along the lateral or longitudinal edges forming the perimeter of the absorbent core and extending substantially parallel to a longitudinal axis (y).

Preferably, the cores herein comprise absorbent material comprising, preferably consisting of, a mixture of superabsorbent polymer particles (SAP) and cellulose fibers (or fluff pulp), typically arranged such that each cluster comprises said mixture. More preferably the absorbent material comprises less than 35%wt, preferably from 5%wt to 30%wt, more preferably from 11%wt to 25%wt of cellulose fibers; and more than 60%wt, preferably more than 65%wt, even more preferably from 70%wt to 95%wt, even more preferably from 75%wt to 85%wt, of SAP by weight of the absorbent material. Advantageously it has been found that unlike fluffless cores (i.e. free of cellulose fibers), such clustered arrangements comprising a mixture with fluff but at the above specified low ranges provides for added speed of liquid distribution and uptake by the SAP whilst still enjoying the benefits of having a considerably thinner core compared to fluff-containing cores of the prior art, this especially without the need for investing in special and costly SAP polymers that would be needed to match said distribution and uptake properties normally otherwise resulting by added gelblocking effects.

THE PROCESS OF MAKING

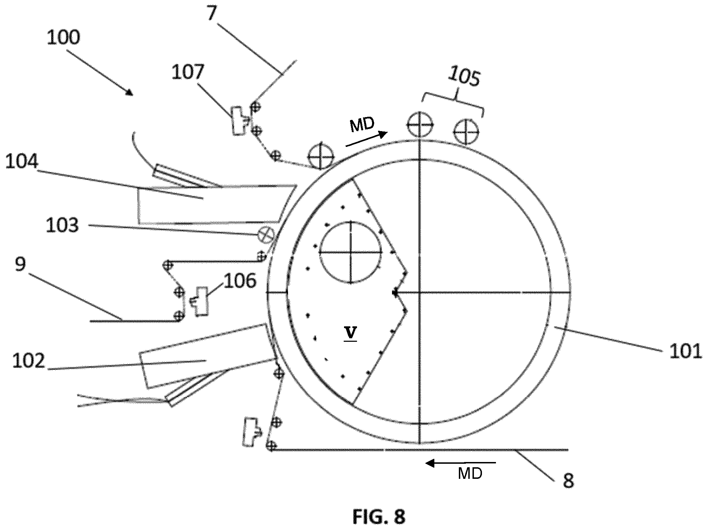

According to an aspect of the disclosure and as exemplified in Fig.8, a method for making an absorbent article comprises the, preferably substantially sequential, steps of:

i. providing a pocket comprising a plurality of porous cavities, wherein said cavities are in fluid communication with an under-pressure source;
ii. providing a first nonwoven web in the form of a bottom layer (8) of a core wrap;
iii. optionally applying an adhesive pattern, preferably in the form of a plurality of adhesive stripes, to said bottom layer (8);
iv. depositing said bottom layer (8) onto said pocket, preferably such that said adhesive pattern faces away from said pocket;
v. depositing a first absorbent material, comprising cellulose fibers and/or superabsorbent polymer particles, over at least a portion of a surface of said bottom layer (8);
vi. optionally applying a second adhesive pattern to an intermediate layer (9);
viii. depositing an intermediate layer (9) over the first absorbent material such that said first absorbent material is sandwiched between said intermediate layer (9) and bottom layer (8);
ix. joining said intermediate layer (9) to said bottom layer (8) to form one or more channel forming areas (10) substantially free of absorbent material and a plurality of first clusters (11') of absorbent material corresponding to said porous cavities;
x. depositing a second absorbent material, comprising cellulose fibers and/or superabsorbent polymer particles, over at least a portion of a said intermediate layer (9);
xi. optionally applying a third adhesive pattern to a second nonwoven web in the form of a top layer (7) of a core wrap;
xii. depositing a second nonwoven web in the form of a top layer (7) of a core wrap over the second absorbent material such that said second absorbent material is sandwiched between said intermediate layer (9) and top layer (7);
xiii. joining said intermediate layer (9) to said top layer (7) to form one or more channel forming areas (10) substantially free of absorbent material and a plurality of second clusters (11") of absorbent material corresponding to said porous cavities, such that an absorbent core is formed comprising said top bottom layer (8), said first absorbent material, said intermediate layer (9), said second absorbent material, and said top layer (7);
xiv. optionally joining an acquisition distribution layer to said absorbent core, typically the body facing surface of said top layer (7), and preferably laminating the absorbent core and the acquisition distribution layer between a liquid pervious topsheet and a liquid impervious backsheet;

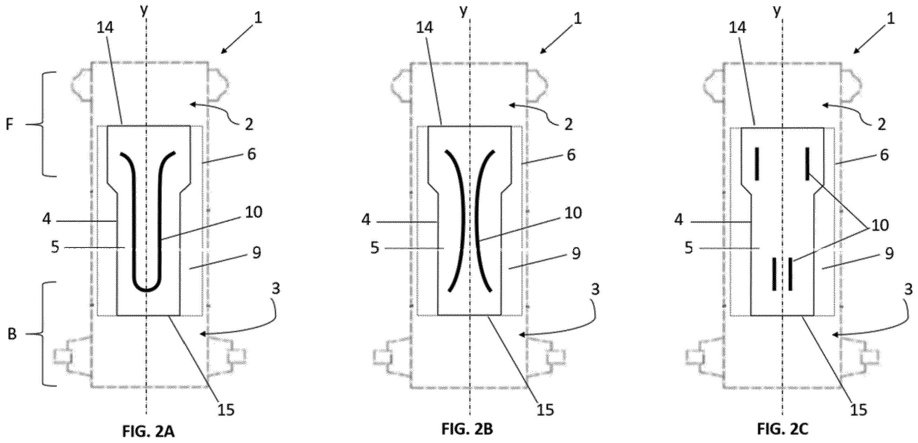

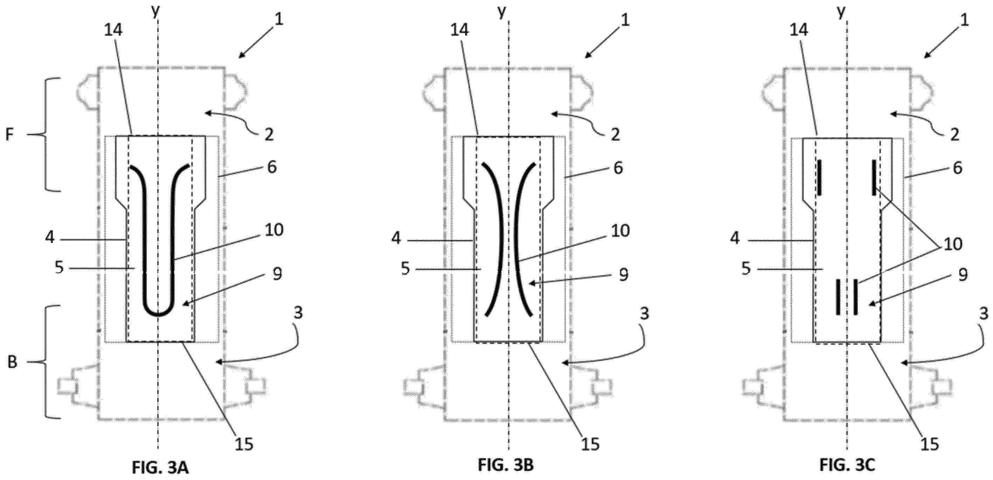

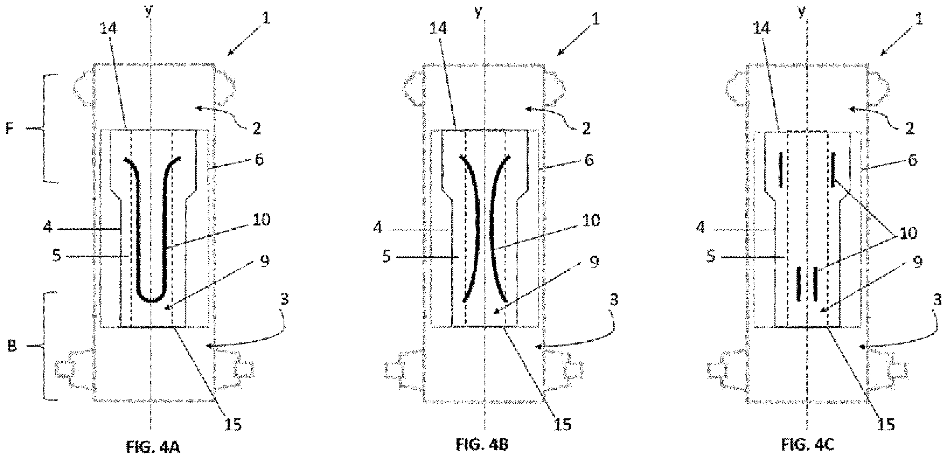

wherein the first plurality of clusters (11') and the second plurality of clusters (11") are substantially congruent or substantially complementary, preferably substantially superpose each other (typically when seen from a planar view generally being a plane formed by the longitudinal axis y and a transverse axis substantially perpendicular thereto as more generally exemplified in Figs. 1-4). Without wishing to be bound by theory, substantially congruent arrangements allow to not only improve core stability but in essence multiply the absorptive retention capacity up to double; whilst substantially complementary (i.e. first plurality of clusters (11') skewed from the second plurality of clusters (11") when seen from a planar view) allow to not only improve core stability but also reduce its overall thickness.

In an embodiment, the first plurality of clusters are mutually separated from the second plurality of clusters by said intermediate layer (9). This arrangement advantageously allows for a separation in the thickness direction that complements the compartments in a width direction to provide additional core stability.

In an embodiment, the method further comprises the step of locally removing the absorbent material applied on said bottom and/or intermediate layers, preferably by a mechanical removal means preferably comprising one or more roller brush or air blower.

In an embodiment, the method further comprises the step of applying a first adhesive pattern on a surface of the intermediate layer facing the first absorbent material; and applying an second adhesive pattern on a surface of the top layer facing the second absorbent material, preferably wherein the first and second patterns are substantially the same or different.

Preferably, the joining step(s) (at least the joining of the intermediate layer (9) to the bottom layer (8)) comprises the step of applying a pressure and/or adhering force to join the first, second, and/or third sheet materials respectively, substantially concurrently with a suction force within the at least one suction zone typically such to combine a downward push with a substantially simultaneous downward pull wherein downward is the direction from the position at which pressure is applied to or towards the supporting member. This is typically achieved by ensuring that the attachment unit(s) herein is/are disposed such to superpose a vacuum region (V) within the support unit (typically in the form of a rotating drum). Advantageously this allows to attain good and strong adhesion without having to apply excessive pressures with the use of e.g. pressure rollers that may inadvertently damage sections of the absorbent core.

In an embodiment, the pattern comprises a plurality, preferably more than 4, even more preferably from 5 to 20, of spaced apart suction zones, typically in the form of distinct cavities, and at least one non-suction zone, such that a plurality of clusters of absorbent material are formed typically after respective application steps.

In an embodiment, the apparatus (100) used to make absorbent articles herein comprises:

> a supporting member (101) for supporting a first sheet material (being the bottom layer (8) along a surface thereof, wherein the surface of said supporting member (101) is provided with at least one suction zone and at least one non-suction zone;
> a first application unit (102) configured for applying a first absorbent material (typically by depositing absorbent material via an airstream, e.g. blowing) on said first sheet material on the supporting member (101); said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone on at least one first attachment portion (A');
a first sheet feed unit configured for applying a second sheet material (being the intermediate layer (9)) on top of the first absorbent material on the first sheet material;
optionally a first attachment unit (103) configured for attaching said first sheet material to said second sheet material at least in the at least one first attachment portion (A');
a second application unit (104) configured for applying a second absorbent material (typically by depositing absorbent material via an airstream, e.g. blowing) on said second sheet material and/or first absorbent material; said second absorbent material being applied such that said second absorbent material is located on a portion of the second sheet material, and wherein substantially no absorbent material is present on other one or more portions of the second sheet material on at least one second attachment portion (A");
a second sheet feed unit configured for applying a third sheet material (being the top layer (7)) on top of the second absorbent material on the second sheet material;
a second attachment unit (105) configured for attaching said second sheet material to said third sheet material (7) at least in the at least one second attachment portion (A");
wherein the first attachment portion (A') and the second attachment portion (A") are substantially congruent or substantially complementary (typically when viewed in a planar view of the absorbent core).

In a preferred embodiment, at least the first application unit (102) (but preferably all the application units described herein) comprises a blowing means (such as an air stream source) to direct the absorbent material onto the bottom layer/first sheet material (8) and/or the intermediate layer/second sheet material (9), and is preferably a non-contact application unit in that it is free of a rotatable laying-out roller comprising a plurality of pockets for applying a spread of absorbent material (also conventionally known or referred to as absorbent material printing). Especially when the absorbent material comprises cellulose fibers it is desirable to apply such absorbent material via a non-contact application in order to achieve good mixing and spacing apart of the superabsorbent polymer particles between cellulose fibers and limit agglomeration of said particles, this allowing to achieve cores with better liquid distribution properties along and across its surface.

In an embodiment, the supporting member (101) is a rotating drum and the at least one non-suction zone comprises at least one elongate zone extending in a circumferential direction of the rotating drum. Preferably, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

In an embodiment, the apparatus further comprises a removing unit comprising a mechanical removal means configured for locally removing the absorbent material applied on said first and/or second sheet material above the at least one non-suction zone. Typically, wherein the mechanical removal means comprises one or more roller brush or air blower.

Preferably, the first sheet feed unit is positioned between the first application unit and the second application unit, and typically upstream of the first attachment unit (103) along a machine direction (MD). Advantageously this allows to ensure reduced risk of contamination between layers.

In an embodiment, the apparatus further comprises a first adhesive unit (106) arranged to apply an adhesive pattern on a surface of the second sheet material facing the first absorbent material; and a second adhesive unit (107) arranged to apply an adhesive pattern on a surface of the third sheet material facing the second absorbent material; said first and second adhesive units being positioned upstream of said first and second attachment units (103, 105) respectively. The apparatus may further comprise a further adhesive unit arranged to apply an adhesive pattern on a surface of the first sheet material facing the first absorbent material.

In an embodiment, the first and second attachment units comprise a pressure means, such as a pressure roller (typically having a substantially smooth contact surface generally such that it is free of protrusions pressing into the channel forming areas so as to limit the need for process registration), arranged to provide an adhering force to join the first, second, and third sheet materials respectively; and preferably wherein the first attachment unit comprises a single pressure means, and the second attachment unit comprises a plurality, preferably from 2 to 5, of pressure means.

In an embodiment, the first attachment unit is positioned between the first application unit and the second application unit, and downstream of the first sheet feed unit, typically along a machine direction (MD). Advantageously this allows to ensure optimal adhesion and reduced risk of contamination between layers.

AUL (Absorbency Under Load, 0.7 psi):

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as WO. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

AUL0.7psig/g=Wb-Wa/Wa-WO

AUL 0.3 psi and 0.5 psi are measured similarly at the appropriate lower pressure.

Absorbtion speed (vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (i.e. that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7.9 mm $\times$ 32 mm, Teflon covered.
4. Stopwatch.
5. Balance, accurate to $\pm$ 0.01 g.
6. Saline solution, 0.9%.
7. Weighing paper.
8. Room with standard condition atmosphere: Temp = 23°C $\pm$ 1°C and Relative

Humidity = 50% ± 2%.

Test procedure:

1. Measure 50 g $\pm$ 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g $\pm$ 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

It is supposed that the present invention is not restricted to any form of realization described previously and that some modifications can be added to the presented example of fabrication without reappraisal of the appended claims.

**Claims**

1. An absorbent article (1) comprising:

   a liquid permeable topsheet (2),
   a liquid impermeable backsheet (3), and
   an absorbent core (4) positioned between said topsheet (2) and backsheet (3),
   wherein the absorbent core (4) comprises an absorbent material (5), said absorbent material comprising cellulose fibers and/or superabsorbent polymers, and wherein said absorbent material is contained within at least one core wrap substrate (6) enclosing said absorbent material therein, wherein the absorbent core further comprises an intermediate layer (9) positioned between a top layer (7) of said core wrap and a bottom layer (8) of said core wrap such that said absorbent material (5) is disposed between said top layer (7) and said intermediate layer (9) and/or between said bottom layer (8) and said intermediate layer (9), and **characterized in that** said top layer (7), said bottom layer (8), and said intermediate layer (9) are joined together to form one or more channel forming areas (10) substantially free of said absorbent material, wherein the intermediate layer (9) is selected from the group consisting of a nonwoven, film, and combinations thereof, and wherein the basis weight of the intermediate layer (9) is greater or equal to the basis weight of the top and/or bottom layers (7, 8) and the intermediate layer (9) has a basis weight of from 15 g/m$^2$ to less than 80 g/m$^2$ and wherein the intermediate layer (9) has a thickness that is greater than, or equal to, the thickness of the top and/or bottom layers (7, 8).

2. An absorbent article according to Claim 1 wherein the intermediate layer (9) is joined, preferably directly, to the top layer (7) and the bottom layer (8) of the core wrap substrate (6) such that said top layer (7) is joined to a body-facing surface of the intermediate layer (9) and the bottom layer (8) is joined to a garment-facing surface of the intermediate layer (9), typically at least within the channel forming areas (10).

3. An absorbent article according to any of the preceding Claims wherein the absorbent core (4) comprises a plurality of clusters (11) of absorbent material (5) wherein more than one, preferably the majority, even more preferably each, of said clusters is circumscribed by said channel forming areas (10), and wherein each said cluster is separated from the neighboring cluster by said channel forming areas (10).

4. An absorbent article according to any of the preceding Claims wherein the top layer (7), the bottom layer (8), and the intermediate layer (9) are joined together by one or more adhesives.

5. An absorbent article according to any of the preceding Claims wherein the top layer (7), the bottom layer (8), and the intermediate layer (9) are joined together by one or more mechanical bonds selected from the group consisting of ultrasonic bonds, thermal bonds, pressure bonds, and combinations thereof.

6. An absorbent article according to any of the preceding Claims wherein the channel forming areas (10) are interconnected such that they extend both along a longitudinal axis (y) and a transversal axis, running substantially perpendicular to the longitudinal axis (y).

7. An absorbent article according to Claims 3 to 6 wherein the absorbent core (4) comprises a first terminal edge cluster (12) and/or a second terminal edge cluster (13) positioned on opposite front and/or back transverse edges (14, 15) of the core respectively, and arranged such that channel(s) formed by the channel forming areas (10) are separated from said front and/or back transverse edges (14, 15) by said first terminal edge cluster (12) and/or said second terminal edge cluster (13) respectively.

8. An absorbent article according to any of the preceding Claims wherein the top layer (7) comprises a first adhesive (17) arranged to define a first adhesive area (A1), the intermediate layer (9) comprises a second adhesive (18) arranged to define a second adhesive area (A2), and the bottom layer (8) comprises a third adhesive (19) arranged to define a third adhesive area (A3), wherein said first adhesive area (A1) and the second adhesive area (A2) are greater than the third adhesive area (A3), and preferably wherein said first, second, and third adhesive areas (A1, A2, A3) comprise one or more adhesive stripes.

9. An absorbent article according to Claim 8 wherein the first adhesive layer (A1) is greater or equal to the second adhesive area (A2), and preferably at least one of said adhesive stripes of the first adhesive area (A1) and the second adhesive area (A2) overlaps said channel forming areas (10), and wherein at least one, preferably at least two, of said adhesive stripes of the third adhesive area (A3) is positioned outboard of said channel forming areas (10).

10. An absorbent article according to Claims 8 to 9 wherein the third adhesive area (A3) consists of a plurality of spaced apart adhesive stripes wherein at least one, preferably at least two, more preferably at least three, even more preferably from 4 to 7, of said adhesive stripes is positioned outboard and/or inboard of the channel forming areas (10).

11. An absorbent article according to Claims 8 to 10 wherein the first and/or second adhesive areas (A1, A2) comprise a substantially uniform single stripe of adhesive arranged to overlap at least a substantial portion of the channel forming areas (10).

12. An absorbent article according to any of the preceding Claims wherein the intermediate layer (9) is a nonwoven selected from the group consisting of wetlaid, airlaid, carded, spunbond, meltblown, carded thermobonded, air-through-bonded, spunlaced, tissue, and combinations thereof, more preferably a nonwoven selected from the group consisting of carded thermobonded, air-through-bonded, spunlaced, and combinations thereof, more preferably a nonwoven selected from the group consisting of air-through-bonded, spunlaced, and combinations thereof, most preferably a spunlaced nonwoven.

13. An absorbent article according to any of the preceding Claims wherein the top layer (7) and bottom layer (8) are the same or different, preferably different, from the intermediate layer (9), and are preferably a nonwoven selected from the group consisting of spunbond, meltblown, carded thermobonded, and combinations thereof.

14. An absorbent article according to any of the preceding Claims wherein the intermediate layer (9) has a width, extending along an axis substantially perpendicular to a longitudinal axis (y), that is less than or equal to a width, extending along an axis substantially perpendicular to a longitudinal axis (y), of the top and/or bottom layers (7,8) of the core wrap.

15. An absorbent article according to Claim 14 wherein the intermediate layer comprises at least one, preferably two, free end (16) that is not joined to the top layer (7) and/or bottom layer (8), preferably two oppositely disposed free ends such that each free end is substantially surrounded by absorbent material, preferably said absorbent material being disposed on a garment facing surface, skin facing surface, and lateral edge, of each free end.

16. An absorbent article according to Claims 14 to 15 wherein the top layer (7) is, preferably directly, joined to the bottom layer (8) at one or more positions being outboard of the intermediate layer (9) and typically at the lateral or longitudinal edges forming the perimeter of the absorbent core and extending substantially parallel to a longitudinal axis (y).

17. An absorbent article according to any of the preceding Claims comprising more than one intermediate layers (9) and wherein the width of each said intermediate layers is preferably less than that of the top and bottom layers (7, 8) such that the top and bottom layers are joined to each other at least along a portion of the perimeter thereof to sandwich said intermediate layers and absorbent material therebetween.

**Patentansprüche**

1. Absorbierender Artikel (1), umfassend:

   eine flüssigkeitsdurchlässige obere Lage (2), eine flüssigkeitsundurchlässige rückseitige Lage (3) und einen absorbierenden Kern (4), der zwischen der oberen Lage (2) und der rückseitigen Lage (3) positioniert ist, wobei der absorbierende Kern (4) ein absorbierendes Material (5) umfasst, das absorbierende Material umfassend Zellulosefasern und/oder superabsorbierende Polymere, und wobei das absorbierende Material innerhalb mindestens eines Kernumhüllungssubstrats (6), das das absorbierende Material darin umschließt, enthalten ist, wobei der absorbierende Kern ferner eine Zwischenschicht (9) umfasst, die zwischen einer oberen Schicht (7) der Kernumhüllung und einer unteren Schicht (8) der Kernumhüllung derart positioniert ist, dass das absorbierende Material (5) zwischen der oberen Schicht (7) und der Zwischenschicht (9) und/oder zwischen der unteren Schicht (8) und der Zwischenschicht (9) angeordnet ist, und **dadurch gekennzeichnet, dass** die obere Schicht (7), die untere Schicht (8) und die Zwischenschicht (9) zusammengefügt sind, um einen oder mehrere kanalbildende Bereiche (10), die im Wesentlichen frei von dem absorbierenden Material sind, zu bilden, wobei die Zwischenschicht (9) aus der Gruppe ausgewählt ist, bestehend aus einem Vlies, einer Folie oder Kombinationen davon, und wobei das Flächengewicht der Zwischenschicht (9) größer als oder gleich dem Flächengewicht der oberen und/oder der unteren Schicht (7, 8) ist, und die Zwischenschicht (9) ein Flächengewicht von 15 g/m$^2$ bis weniger als 80 g/m$^2$ aufweist, und wobei die Zwischenschicht (9) eine Dicke, die größer als oder gleich der Dicke der oberen und/oder der unteren Schicht (7, 8) ist, aufweist.

2. Absorbierender Artikel nach Anspruch 1, wobei die Zwischenschicht (9), vorzugsweise direkt, mit der oberen Schicht (7) und der unteren Schicht (8) des Kernumhüllungssubstrats (6) derart zusammengefügt ist, dass die obere Schicht (7) mit einer körperzugewandten Oberfläche der Zwischenschicht (9) zusammengefügt ist und die untere Schicht (8) mit einer kleidungsstückzugewandten Oberfläche der Zwischenschicht (9), üblicherweise mindestens innerhalb der kanalbildenden Bereiche (10), zusammengefügt ist.

3. Absorbierender Artikel nach einem der vorstehenden Ansprüche, wobei der absorbierende Kern (4) eine Vielzahl von Clustern (11) aus absorbierendem Material (5) umfasst, wobei mehr als ein, vorzugsweise die Mehrheit, noch mehr bevorzugt jeder der Cluster, durch die kanalbildenden Bereiche (10) umbeschrieben ist, und wobei jeder der Cluster durch die kanalbildenden Bereiche (10) von den benachbarten Clustern getrennt ist.

4. Absorbierender Artikel nach einem der vorstehenden Ansprüche, wobei die obere Schicht (7), die untere Schicht (8) und die Zwischenschicht (9) durch einen oder mehrere Klebstoffe zusammengefügt sind.

5. Absorbierender Artikel nach einem der vorstehenden Ansprüche, wobei die obere Schicht (7), die untere Schicht (8) und die Zwischenschicht (9) durch eine oder mehrere mechanische Bindungen, die aus der Gruppe ausgewählt sind, bestehend aus Ultraschallbindungen, thermischen Bindungen, Druckbindungen und Kombinationen davon, zusammengefügt sind.

6. Absorbierender Artikel nach einem der vorstehenden Ansprüche, wobei die kanalbildenden Bereiche (10) derart miteinander verbunden sind, dass sie sich sowohl entlang einer Längsachse (y) als auch einer Querachse, die im Wesentlichen zu der Längsachse (y) senkrecht verläuft, erstrecken.

7. Absorbierender Artikel nach den Ansprüchen 3 bis 6, wobei der absorbierende Kern (4) einen ersten Endkantencluster (12) und/oder einen zweiten Endkantencluster (13) umfasst, der jeweils an gegenüberliegenden vorderen und/oder hinteren Querkanten (14, 15) des Kerns positioniert ist und derart arrangiert ist, dass ein Kanal/Kanäle, der/die durch die kanalbildenden Bereiche (10) gebildet werden, jeweils durch den ersten Endkantencluster (12) und/oder den zweiten Endkantencluster (13) von den vorderen und/oder hinteren Querkanten (14, 15) getrennt sind.

8. Absorbierender Artikel nach einem der vorstehenden Ansprüche, wobei die obere Schicht (7) einen ersten Klebstoff (17), der arrangiert ist, um einen ersten Klebstoffbereich (A1) zu definieren, umfasst, die Zwischenschicht (9) einen zweiten Klebstoff (18), der arrangiert ist, um einen zweiten Klebstoffbereich (A2) zu definieren, umfasst und die untere Schicht (8) einen dritten Klebstoff (19), der arrangiert ist, um einen dritten Klebstoffbereich (A3) zu definieren, umfasst, wobei der erste Klebstoffbereich (A1) und der zweite Klebstoffbereich (A2) größer als der dritte Klebstoffbereich (A3) sind, und vorzugsweise wobei der erste, der zweite und der dritte Klebstoffbereich (A1, A2, A3) einen oder mehrere Klebstoffstreifen umfassen.

9. Absorbierender Artikel nach Anspruch 8, wobei die erste Klebstoffschicht (A1) größer als oder gleich dem zweiten Klebstoffbereich (A2) ist und vorzugsweise mindestens einer der Klebstoffstreifen des ersten Klebstoffbereichs (A1) und des zweiten Klebstoffbereichs (A2) die kanalbildenden Bereiche (10) überlappt, und wobei mindestens einer, vorzugsweise mindestens zwei, der Klebstoffstreifen des dritten Klebstoffbereichs (A3) außerhalb der kanalbildenden Bereiche (10) positioniert ist.

10. Absorbierender Artikel nach den Ansprüchen 8 bis 9, wobei der dritte Klebstoffbereich (A3) aus einer Vielzahl von beabstandeten Klebstoffstreifen besteht, wobei mindestens einer, vorzugsweise mindestens zwei, mehr bevorzugt mindestens drei, noch mehr bevorzugt von 4 bis 7 der Klebstoffstreifen außerhalb und/oder innerhalb der kanalbildenden Bereiche (10) positioniert ist.

11. Absorbierender Artikel nach den Ansprüchen 8 bis 10, wobei der erste und/oder der zweite Klebstoffbereich (A1, A2) einen im Wesentlichen gleichmäßigen einzelnen Klebstoffstreifen, der arrangiert ist, um mindestens einen wesentlichen Abschnitt der kanalbildenden Bereiche (10) zu überlappen, umfasst.

12. Absorbierender Artikel nach einem der vorstehenden Ansprüche, wobei die Zwischenschicht (9) ein Vlies, das aus der Gruppe ausgewählt ist, bestehend aus nassgelegt, luftgelegt, kardiert, spunbond, schmelzgeblasen, kardiert thermisch gebunden, air-through-bonded, spunlaced, Gewebe und Kombinationen davon, mehr bevorzugt ein Vlies, das aus der Gruppe ausgewählt ist, bestehend aus kardiert thermisch gebunden, air-through-bonded, spunlaced und Kombinationen davon, am meisten bevorzugt ein Spunlaced-Vlies, ist.

13. Absorbierender Artikel nach einem der vorstehenden Ansprüche, wobei die obere Schicht (7) und die untere Schicht (8) mit der Zwischenschicht (9) gleich oder verschieden, vorzugsweise verschieden von ihr, sind und vorzugsweise ein Vlies sind, das aus der Gruppe ausgewählt ist, bestehend aus spunbond, schmelzgeblasen, kardiert thermisch gebunden und Kombinationen davon.

14. Absorbierender Artikel nach einem der vorstehenden Ansprüche, wobei die Zwischenschicht (9) eine Breite, die sich entlang einer Achse, die im Wesentlichen zu einer Längsachse (y) senkrecht ist, erstreckt, die kleiner als oder gleich einer Breite, die sich entlang einer Achse, die im Wesentlichen zu einer Längsachse (y) senkrecht ist, erstreckt, der oberen und/oder der unteren Schicht (7,8) der Kernumhüllung aufweist.

15. Absorbierender Artikel nach Anspruch 14, wobei die Zwischenschicht mindestens ein, vorzugsweise zwei, freies Ende (16), das nicht mit der oberen Schicht (7) und/oder der unteren Schicht (8) zusammengefügt ist, vorzugsweise zwei gegenüberliegend angeordnete freie Enden, derart, dass jedes freie Ende im Wesentlichen durch absorbierendes Material umgeben ist, umfasst, vorzugsweise wobei das absorbierende Material auf einer kleidungsstückzugewandten Seite, einer hautzugewandten Seite und einer seitlichen Kante jedes freien Endes angeordnet ist.

16. Absorbierender Artikel nach den Ansprüchen 14 bis 15, wobei die obere Schicht (7), vorzugsweise direkt, mit der unteren Schicht (8) an einer oder mehreren Positionen, die außerhalb der Zwischenschicht (9) sind, und üblicherweise an den seitlichen oder Längskanten, die den Umfang des absorbierenden Kerns bilden und sich im Wesentlichen zu einer Längsachse (y) parallel erstrecken, zusammengefügt ist.

17. Absorbierender Artikel nach einem der vorstehenden Ansprüche, umfassend mehr als eine Zwischenschicht (9), und wobei die Breite jeder der Zwischenschichten vorzugsweise kleiner als die der oberen und der unteren Schicht (7, 8) ist, derart, dass die obere und die untere Schicht mindestens entlang eines Abschnitt des Umfangs davon zusammengefügt sind, um die Zwischenschichten und das absorbierende Material dazwischen einzuschieben.

## Revendications

1. Article absorbant (1) comprenant :

une feuille de dessus (2) perméable aux liquides,
une feuille de fond (3) imperméable aux liquides, et
une âme absorbante (4) placée entre ladite feuille de dessus (2) et ladite feuille de fond (3), dans lequel l'âme absorbante (4) comprend un matériau absorbant (5), ledit matériau absorbant comprenant des fibres de cellulose et/ou des polymères superabsorbants, et dans lequel ledit matériau absorbant est contenu au sein d'au moins un substrat d'enveloppe d'âme (6) renfermant ledit matériau absorbant à l'intérieur de celui-ci, dans lequel l'âme absorbante comprend en outre une couche intermédiaire (9) positionnée entre une couche de dessus (7) de ladite enveloppe d'âme et une couche de dessous (8) de ladite enveloppe d'âme de telle sorte que ledit matériau absorbant (5) est disposé entre ladite couche de dessus (7) et ladite couche intermédiaire (9) et/ou entre ladite couche de dessous (8) et ladite couche intermédiaire (9), et **caractérisé en ce que** ladite couche de dessus (7), ladite couche de dessous (8), et ladite couche intermédiaire (9) sont jointes ensemble pour former une ou plusieurs zones de formation de canaux (10) essentiellement exemptes dudit matériau absorbant, dans lequel la couche intermédiaire (9) est choisie dans le groupe constitué d'un non-tissé, d'un film, et de combinaisons de ceux-ci, et dans lequel le poids de base de la couche intermédiaire (9) est supérieur ou égal au poids de base des couches de dessus et/ou de dessous (7, 8) et la couche intermédiaire (9) a un poids de base allant de 15 g/m$^2$ à moins de 80 g/m$^2$ et dans lequel la couche intermédiaire (9) a une épaisseur supérieure à, ou égale à, l'épaisseur des couches de dessus et/ou de dessous (7, 8).

2. Article absorbant selon la revendication 1 dans lequel la couche intermédiaire (9) est jointe, de préférence directement, à la couche de dessus (7) et à la couche de dessous (8) du substrat d'enveloppe d'âme (6) de telle sorte que ladite couche de dessus (7) est jointe à une surface faisant face vers le corps de la couche intermédiaire (9) et la couche de dessous (8) est jointe à une surface faisant face vers le vêtement de la couche intermédiaire (9), de manière générale au moins au sein des zones de formation de canaux (10).

3. Article absorbant selon l'une quelconque des revendications précédentes dans lequel l'âme absorbante (4) comprend une pluralité de grappes (11) de matériau absorbant (5) dans lequel plus d'une, de préférence la majorité, encore plus préférablement chacune, desdites grappes est circonscrite par lesdites zones de formation de canaux (10), et dans lequel chaque dite grappe est séparée de la grappe voisine par lesdites zones de formation de canaux (10).

4. Article absorbant selon l'une quelconque des revendications précédentes dans lequel la couche de dessus (7), la couche de dessous (8), et la couche intermédiaire (9) sont jointes ensemble par un ou plusieurs adhésifs.

5. Article absorbant selon l'une quelconque des revendications précédentes dans lequel la couche de dessus (7), la couche de dessous (8), et la couche intermédiaire (9) sont jointes ensemble par une ou plusieurs liaisons mécaniques choisies dans le groupe constitué de liaisons par ultrasons, liaisons thermiques, liaisons par pression, et des combinaisons de celles-ci.

6. Article absorbant selon l'une des revendications précédentes dans lequel les zones de formation de canaux (10) sont interconnectées de telle sorte qu'elles s'étendent à la fois le long d'un axe longitudinal (y) et d'un axe transversal, s'inscrivant essentiellement perpendiculairement à l'axe longitudinal (y).

7. Article absorbant selon les revendications 3 à 6 dans lequel l'âme absorbante (4) comprend une première grappe de bords terminaux (12) et/ou une seconde grappe de bords terminaux (13) positionnées sur des bords transversaux opposés avant et/ou arrière (14, 15) de l'âme respectivement, et agencées de telle sorte qu'un ou plusieurs canaux formés par les zones de formation de canaux (10) sont séparés desdits bords transversaux avant et/ou arrière (14, 15) par ladite première grappe de bords terminaux (12) et/ou ladite seconde grappe de bords terminaux (13) respectivement.

8. Article absorbant selon l'une quelconque des revendications précédentes dans lequel la couche de dessus (7) comprend un premier adhésif (17) agencé pour définir une première zone d'adhésif (A1), la couche intermédiaire (9) comprend un deuxième adhésif (18) agencé pour définir une deuxième zone d'adhésif (A2), et la couche de dessous (8) comprend un troisième adhésif (19) agencé pour définir une troisième zone d'adhésif (A3), dans lequel ladite première zone d'adhésif (A1) et la deuxième zone d'adhésif (A2) sont supérieures à la troisième zone d'adhésif (A3), et de préférence dans lequel lesdites première, deuxième, et troisième zones d'adhésif (A1, A2, A3) comprennent une ou plusieurs bandes d'adhésif.

9. Article absorbant selon la revendication 8 dans lequel la première couche d'adhésif (A1) est supérieure ou égale à la deuxième zone d'adhésif (A2), et de préférence au moins l'une desdites bandes d'adhésif de la première zone d'adhésif (A1) et de la deuxième zone d'adhésif (A2) chevauche lesdites zones de formation de canaux (10), et dans lequel au moins l'une, de préférence au moins deux, desdites bandes d'adhésif de la troisième zone d'adhésif (A3) sont positionnées à l'extérieur desdites zones de formation de canaux (10).

10. Article absorbant selon les revendications 8 à 9 dans lequel la troisième zone d'adhésif (A3) est constituée d'une pluralité de bandes d'adhésif espacées dans lequel au moins l'une, de préférence au moins deux, plus préférablement au moins trois, encore plus préférablement de 4 à 7, desdites bandes d'adhésif sont positionnées à l'extérieur et/ou à l'intérieur des zones de formation de canaux (10).

**11.** Article absorbant selon les revendications 8 à 10 dans lequel les première et/ou deuxième zones d'adhésif (A1, A2) comprennent une bande unique essentiellement uniforme d'adhésif agencée pour chevaucher au moins une partie essentielle des zones de formation de canaux (10).

**12.** Article absorbant selon l'une quelconque des revendications précédentes dans lequel la couche intermédiaire (9) est un non-tissé choisi dans le groupe constitué de non-tissé par voie humide, par voie aérienne, cardé, par filage direct, fondu-soufflé, cardé thermolié, lié par traversée d'air, lacé par filage, tissu, et des combinaisons de ceux-ci, plus préférablement un non-tissé choisi dans le groupe constitué de cardé thermolié, lié par traversée d'air, lacé par filage, et des combinaisons de ceux-ci, plus préférablement un non-tissé choisi dans le groupe constitué de lié par traversée d'air, lacé par filage, et des combinaisons de ceux-ci, le plus préférablement un non-tissé lacé par filage.

**13.** Article absorbant selon l'une quelconque des revendications précédentes dans lequel la couche de dessus (7) et la couche de dessous (8) sont les mêmes que ou différentes, de préférence différentes, de la couche intermédiaire (9), et sont de préférence un non-tissé choisi dans le groupe constitué de non-tissé par filage direct, fondu-soufflé, cardé thermolié, et des combinaisons de ceux-ci.

**14.** Article absorbant selon l'une quelconque des revendications précédentes dans lequel la couche intermédiaire (9) a une largeur, s'étendant le long d'un axe essentiellement perpendiculaire à un axe longitudinal (y), qui est inférieure ou égale à une largeur, s'étendant le long d'un axe essentiellement perpendiculaire à un axe longitudinal (y), des couches de dessus et/ou de dessous (7,8) de l'enveloppe d'âme.

**15.** Article absorbant selon la revendication 14 dans lequel la couche intermédiaire comprend au moins une, de préférence deux, extrémités libres (16) qui ne sont pas jointes à la couche de dessus (7) et/ou à la couche de dessous (8), de préférence deux extrémités libres disposées de manière opposée de telle sorte que chaque extrémité libre est essentiellement entourée de matériau absorbant, de préférence ledit matériau absorbant étant disposé sur une surface faisant face vers le vêtement, une surface faisant face vers la peau, et un bord latéral, de chaque extrémité libre.

**16.** Article absorbant selon les revendications 14 à 15 dans lequel la couche de dessus (7) est, de préférence directement, jointe à la couche de dessous (8) au niveau d'une ou plusieurs positions étant à l'extérieur de la couche intermédiaire (9) et de manière générale au niveau des bords latéraux ou longitudinaux formant le périmètre de l'âme absorbante et s'étendant essentiellement parallèlement à un axe longitudinal (y).

**17.** Article absorbant selon l'une quelconque des revendications précédentes comprenant plus d'une couche intermédiaire (9) et dans lequel la largeur de chaque dite couche intermédiaire est de préférence inférieure à celle des couches de dessus et de dessous (7, 8) de telle sorte que les couches de dessus et de dessous sont jointes l'une à l'autre au moins le long d'une partie du périmètre de celles-ci pour prendre en sandwich lesdites couches intermédiaires et ledit matériau absorbant entre elles.

EP 4 082 496 B1

FIG. 1A

FIG. 1B

FIG. 1C

FIG. 1D

19

FIG. 2A

FIG. 2B

FIG. 2C

**FIG. 3A**

**FIG. 3B**

**FIG. 3C**

EP 4 082 496 B1

FIG. 4A

FIG. 4B

FIG. 4C

22

FIG. 5C

FIG. 5B

FIG. 5A

**FIG. 6**

FIG. 7A

FIG. 7B

FIG. 7C

FIG. 7D

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012170778 A, Rosati
- WO 2012170779 A
- WO 2012170781 A
- WO 20121708008 A
- EP 3342386 A
- WO 2018172860 A
- EP 3453368 A
- EP 2679210 A1
- US 3485706 A, Evans
- US 20090306290 A